# EUROPÄISCHE PATENTANMELDUNG

(11) **EP 0 908 188 A2**
(43) Veröffentlichungstag der Anmeldung: **14.04.1999**
(21) Anmeldenummer: 98118260.3
(22) Anmeldetag: 26.09.1998
(51) Int. Cl.: A61L 9/01, C11D 17/00

(54) **Toilettensteine**

(30) Priorität: 09.10.1997 DE 19744571
(71) Anmelder: HAARMANN & REIMER GMBH, D-37601 Holzminden (DE)
(72) Erfinder: Wrede, Wolfgang, 37603 Holzminden (DE); Lammert, Matthias, 59590 Geseke (DE)
(74) Vertreter: Mann, Volker, Dr. (DE)

(57) **Zusammenfassung**

Die Erfindung stellt Toilettensteine bereit, die während Zeiten des Nichtspülens Riechstoffe an die Umgebung und während der Spülvorgänge Reinigungsmittel an das Wasser abgeben.

## Beschreibung

Die Erfindung betrifft eine stückige Zubereitung, die zur Geruchsverbesserung und Reinigung in Toilettenbecken eingesetzt werden kann.

Toilettensteine werden mittels geeigneter Behälter in Toilettenbecken gehängt; sie werden deshalb oft 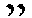 rim blocks" genannt. Sie geben ihre Inhaltsstoffe vor allem an das Spülwasser ab. Obwohl Toilettensteine ihre Duftstoffe an die umgebende Luft abgeben sollten, wird der größte Teil der Riechstoffe durch das Spülwasser wegtransportiert, ohne daß diese Riechstoffe den ihnen zugedachten Zweck erfüllt hätten.

Die Aufgabe der Erfindung bestand darin, ein Produkt bereitzustellen, dessen Riechstoffe besser genutzt werden. Das Produkt soll nicht flüssig sein und nicht merklich quellen.

Es wurde nun gefunden, daß die Aufgabe durch eine Zubereitung folgender Zusammensetzung gelöst werden kann:
a) 1 bis 10 Gew.-Teile Riechstoff,
b) 2 bis 20 Gew.-Teile C₁₀-C₂₀-Fettsäure-Na-salz,
c) 10 bis 40 Gew.-Teile organisches Lösungsmittel aus der Reihe C₁-C₆-Alkanol, C₂-C₆-Alkandiol und deren Mischungen,
d) 25 bis 60 Gew.-Teile Wasser,
e) 15 bis 25 Gew.-Teile anionisches und/oder nicht-ionisches Tensid und gegebenenfalls
f) bis zu 20 Gew.-Teile Salz (mindestens) einer Mineralsäure,
wobei sich die Gewichtsangaben jeweils auf 100 Gewichtsteile der Summe der Komponenten a) bis d) beziehen.

Die Riechstoffe a) bestehen in der Regel aus einer Vielzahl von Komponenten. Die Art der Riechstoffe richtet sich nach der Verträglichkeit mit den anderen Komponenten der Zusammensetzung und des gewünschten Geruchs. Der Fachmann kann aus einer Vielzahl von Gerüchen die von ihm bevorzugte Note auswählen.

Die Natriumsalze von C₁₀-C₂₀-Fettsäuren b) umfassen vorzugsweise C₁₂-C₁₈-Fettsäure-Na-Salze und deren Mischungen, u. a. Natriumpalmitat und Natriumstearat sowie deren Mischungen.

Die C₁-C₆-Alkanole c) umfassen Methanol, Ethanol, n- und iso-Propanol, n-, iso- und tert.-Butanol, n-Pentanol und n-Hexanol. Die C₂-C₆-Alkandiole c) umfassen Ethylenglykol, Propandiol-1,2 und -1,3, Butandiole und Hexandiole. Unter die Alkohole c) fallen auch solche Verbindungen, deren Ketten durch ein Ether-Sauerstoffatom unterbrochen sind, wie z.B. Diethylenglykol, Dipropylenglykol, 2-Methoxymethanol, 2-Ethoxyethanol, 2-Butoxyethanol und 2-(2-Ethoxyethoxy)-ethanol.

Die anionischen und nicht-ionischen Tenside e) umfassen vor allem sulfonierte ethoxylierte C₁₂-C₁₈-Alkohole wie z.B. Fettalkoholethersulfate, wie z.B. sulfonierte ethoxylierte Kokosfettalkohole (C₁₂-C₁₈, vorzugsweise C₁₂-C₁₄-Fettalkohole) wie ®Texapan N 28 (Henkel), ®Marlinat 242/28 (Hüls), ®Genapol LRO flüssig (Hoechst), ethoxylierten hydrierten Ricinusfettalkohol, α-Sultofettsäuremethylester sowie C₁₂-C₁₈-Alkyldimethylaminoxide.

Als Salze von Mineralsäuren kommen z.B. Kochsalz, Natriumsulfat etc. in Frage. Durch Zugabe von Elektrolyt werden die herrschenden Abstoßungskräfte der geladenen Micellen reduziert, so daß der mittlere Abstand zwischen den Micellen kleiner wird. Die für die Gelierung entscheidende Wechselwirkung zwischen den Micellen wird somit verstärkt und die Gelierzeit verkürzt. Gleichzeitig wird der Schmelzpunkt erhöht.

Toilettensteine dienen neben der Geruchsverbesserung auch gleichzeitig zur Reinigung bzw. zur Verhinderung von Verkrustungsbildung und zur Desinfektion. Sie können deshalb neben Riechstoffen auch Tenside, Säurekomponenten und Mikrobizide enthalten.

Aus der US-PS 4 666 671 sind bereits Toilettensteine bekannt, die
a) C₁₀-C₂₀-Fettsäure-Na-salze, Natriumalginat, Carboxymethylcellulose, Carageenan, Hydroxypropylcellulose, Stärke oder Gummis,
b) organisches Lösungsmittel,
c) Wasser und
d) Riechstoffe enthalten.

Obwohl die US-PS 4 666 671 also schon gleiche oder ähnliche Komponenten beschrieben hat, unterscheiden sich die Mengen erheblich: So enthalten die Produkte der US-PS 4 666 671 - anders als gemäß unserer Erfindung - mindestens dreimal soviel organisches Lösungsmittel wie Wasser. Es erscheint erstaunlich, daß unsere Zubereitungen, die einen größeren Wasseranteil enthalten, die Spülvorgänge überstehen, ohne sich vorschnell aufzulösen oder zu krümeln. Die erfindungsgemäßen Toilettensteine stellen eine glückliche Kombination wünschenswerter Eigenschaften bereit; insbesondere lassen sie eine permanente Verdampfung der Riechstoffe zu, auch wenn längere Zeit nicht gespült wird, und sie gestatten eine Abgabe der für die Reinigung der Toilettenschüsseln notwendigen Tenside bei jedem Spülvorgang.

Die erfindungsgemäßen Toilettensteine können weitere Zusätze, wie z.B. Transparenzverbesserer, Schaumverstärker und ähnliche Hilfsmittel enthalten.

Die Herstellung der Toilettensteine kann durch einfaches Mischen der Komponenten, durch Gießen der erhaltenen Mischungen und durch Zerkleinern der erstarrten Formkörper auf ein geeignetes Maß erfolgen. Man kann die Mischungen auch in Behälter gießen, die der Endverbraucher direkt einsetzen kann, und dort erstarren lassen.

Die nachfolgenden Beispiele erläutern die Erfindung. Prozentangaben beziehen sich jeweils auf das Gewicht.

### Beispiele

### Beispiel 1

- 25,0 %: 1,2-Propandiol,
- 25,0 %: Wasser,
- 18,0 %: einer 28 %igen wäßrigen Natriumlaurylethersulfat-Lösung (sulfonierter, ethoxylierter Kokosfettalkohol),
- 12,0 %: Talgfettalkoholpolyglykolether (25 mol Ethylenoxideinheiten),
- 3,0 %: Natriumstearat,
- 4,0 %: Natriumpalmitat,
- 5,0 %: Ethanol,
- 3,0 %: hydrierter ethoxylierter Ricinusfettalkohol und
- 5,0 %: Parfumöl.

Aus der obigen Mischung wurden Toilettensteine (Sticks) hergestellt. Sie besaßen einen Schmelzpunkt von ca. 55°C, waren transparent und recht hart.

Die hergestellten Blöcke wurden maschinell und manuell in verschiedenen Zeitintervallen abgespült. Nach Trocknung der Oberflächen wurden die Sticks gewogen. Parallel dazu wurde ein Stick der gleichen Serie offen gelagert und ebenfalls die Gewichtsabnahme bestimmt.

Die gravimetrischen Bestimmungen ergaben beim Abspülverhalten eine Lebensdauer von 550 Spülmengen bei einer 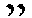 Halbwertszeit" bei 210 Spülungen und bei der offenen Lagerung Verdampfungsverluste von 30 % innerhalb von 14 Tagen und von 40 % innerhalb von 3 Wochen.

### Beispiel 2

- 25,0 %: einer 28 %igen wäßrigen Natriumlaurylethersulfat-Lösung,
- 24,0 %: einer 30 %igen wäßrigen Alkyldimethylaminoxid-Lösung,
- 4,0 %: Parfüm,
- 4,0 %: hydrierter ethoxylierter Ricinusfettalkohol,
- 12,0 %: Propylenglykol,
- 14,8 %: Wasser,
- 11,0 %: Kochsalz und
- 5,2 %: Natriumstearat.

## Patentansprüche

1. Zubereitung zur Geruchsverbesserung und Reinigung in Toilettenbecken enthaltend
a) 1 bis 10 Gew.-Teile Riechstoff,
b) 2 bis 20 Gew.-Teile C₁₀-C₂₀-Fettsäure-Na-salz,
c) 10 bis 40 Gew.-Teile organisches Lösungsmittel aus der Reihe C₁-C₆-Alkanol, C₂-C₆-Alkandiol und deren Mischungen,
d) 25 bis 60 Gew.-Teile Wasser,
e) 15 bis 25 Gew.-Teile anionisches und/oder nicht-ionisches Tensid und gegebenenfalls
f) bis zu 20 Gew.-Teile Salz (mindestens) einer Mineralsäure,
wobei sich die Gewichtsangaben jeweils auf 100 Gewichtsteile der Summe der Komponenten a) bis d) beziehen.
